Europäisches Patentamt

(19) European Patent Office

Office européen des brevets

(11) Publication number: **0 181 127 B1**

## EUROPEAN PATENT SPECIFICATION

(45) Date of publication of patent specification: 27.02.91

(51) Int. Cl.⁵: **C07C  63/26**, C07C  51/42, C07C  51/44, C07C  51/48

(21) Application number: 85307705.5

(22) Date of filing: 24.10.85

(54) **Recovery of metal catalyst residues and other useful products from therephthalic acid production.**

(30) Priority: 06.11.84 GB 8428028

(43) Date of publication of application:
14.05.86 Bulletin  86/20

(45) Publication of the grant of the patent:
27.02.91 Bulletin  91/09

(84) Designated Contracting States:
BE DE FR GB IT

(56) References cited:
FR-A- 2 162 572
GB-A- 1 413 488
GB-A- 1 595 974
GB-A- 2 067 563
US-A- 3 780 096

(73) Proprietor: IMPERIAL CHEMICAL INDUSTRIES PLC
Imperial Chemical House, Millbank
London SW1P 3JF(GB)

(72) Inventor: Corry, Alfred Reginald
102 Marwood Drive
Great Ayton North Yorkshire(GB)
Inventor: McGregor, Gordon
7 Springfield Avenue
Stockton on Tees Cleveland(GB)
Inventor: Royall, David John
59 Farndale Drive
Guisborough Cleveland(GB)

(74) Representative: Locke, Timothy John et al
Imperial Chemical Industries PLC Legal Department: Patents Bessemer Road PO Box 6
Welwyn Garden City Hertfordshire, AL7 1HD(GB)

## Description

This invention relates to the recovery of catalyst residues and other useful products from terephthalic acid production.

Terephthalic acid is commercially produced by the oxidation of para-xylene with molecular oxygen in a solution of cobalt and manganese compounds and bromine in acetic acid. The cobalt and manganese compounds and bromine act as catalysts. The oxidation may take place in one or more stages and is usually carried out continuously, the product from the first stage being re-treated with oxygen in at least one subsequent stage in order substantially to complete the oxidation. Terephthalic acid separates from the reaction medium and so may be removed by conventional means, for example centrifuging or filtering, leaving a mother liquor containing the catalyst and by-products of the reaction, which may be recycled to the reaction optionally after treatment for example to remove water.

In order to prevent a build-up of by-products in the process part of the mother liquor is normally purged from the process and the reactor feed made up with fresh catalyst and acetic acid. The purged mother liquor may be distilled to recover acetic acid from it and the residue may of course be discarded, and for example burnt, but it is desirable to recover the catalyst metals from it as both cobalt and manganese are expensive elements.

It is known from Japanese specification 53/114,796 to extract with water and to recover the metals with an ion exchange resin.

UK patent specification 1,413,488 discloses a process in which cobalt and manganese are recovered from residues from terephthalic acid production which contain iron by treating them with water and an alkali metal carbonate to dissolve acidic organic materials, separating a metal carbonate precipitate, dissolving the metal carbonate in excess acetic acid and distilling the excess acetic acid whereby an insoluble form of iron is precipitated.

In US patent specification 3,780,096 the recovery of cobalt and manganese oxidation catalyst from a solution resulting from the oxidation of aliphatic substituted aromatic hydrocarbons to carboxylic acids by the addition of a mineral acid to precipitate the catalyst as a filterable salt is disclosed, and the process is exemplified in the oxidation of mono methyl naphthalenes to naphthoic acids.

We have now devised a process in which not only are the metal catalyst residues recovered substantially completely but in which other useful products are recovered also.

The invention comprises a process of producing terephthalic acid in which para-xylene is oxidised to terephthalic acid by contacting it in solution in acetic acid with elemental oxygen in the presence of a dissolved catalyst which comprises cobalt, manganese and preferably bromine, separating terephthalic acid as a solid from a mother liquor, distilling acetic acid from at least part of the mother liquor to leave a residue, dissolving the alkali soluble part of the residue in an aqueous solution of an ammonium or alkali metal hydroxide, carbonate or bicarbonate and thereby leaving a solid deposit of cobalt and manganese compounds, separating the solid deposit, precipitating organic acids by adding acid to the aqueous solution, and separating them, solvent extracting the organic acids to remove the monobasic acids and leaving a residue comprising mixed polybasic organic acids.

If desired the mother liquor as aforesaid may be distilled to leave a residue comprising some of the acetic acid and water as well as catalyst residues and aromatic acids. If desired the residues may be washed with water to remove some of the catalyst before the alkali soluble part is dissolved in the said aqueous solution.

The solid deposit of cobalt and manganese compounds may be readily reacted with hydrobromic acid to produce a solution sufficiently pure for return to the oxidation step of the process as catalyst. It is believed that the solid deposit comprises oxides, hydroxides and/or carbonates of cobalt and manganese.

The acids precipitated from the alkaline solution are suitably washed with water to remove water soluble salts and are then extracted with a solvent in which benzoic acid has a high solubility and polybasic acids a much lower solubility. Suitable solvents may be selected on these criteria from works of reference or relevant data may be determined experimentally. Suitable solvents include for example benzene and alkyl benzenes having a total of 1 to 4 carbon atoms in their alkyl groups and preferably toluene, diethyl and preferably dimethyl succinate, glutarate and/or adipate, butoxy, propoxy or ethoxy ethanol, or suitable chlorohydrocarbon solvents for example carbon tetrachloride, chloroform or 1,1,1,-trichloroethane or ethylene glycol or mixtures thereof. If other monobasic acids are present they tend to be removed with the benzoic acid; if due to incomplete oxidation of para-xylene in the process paratoluic acid is present this therefore is removed also. If necessary the benzoic acid may be purified for example by recrystallisation, but for certain purposes, for example use in motor vehicle antifreeze compositions this may be unnecessary.

The mixed polybasic organic acids remaining after the substantial removal of the benzoic and other monobasic acids are useful as intermediates for resin manufacture.

## EXAMPLE

A waste by-product stream in the form of a slurry obtained by oxidising para-xylene to terephthalic acid by contacting it in solution in acetic acid with elemental oxygen in the presence of catalyst which comprises cobalt, manganese and bromine, and removing solid terephthalic acid, distilling part of the mother liquor to remove some acetic acid (the remaining mother liquor being recycled) (3000 parts) contained in approximate proportions, mixed aromatic carboxylic acids (1620 parts), metallic catalyst residues (30 parts) in a ratio by weight of the elements manganese/cobalt, 2/1, acetic acid (550 parts) and water (845 parts). It was diluted by agitating with cold water (3000 parts) and neutralised with 47% aqueous caustic soda (2550 parts).

The resulting mobile liquid containing a small amount of suspended solids was cooled and filtered. The grey-black solid collected on the filter was washed once with cold water (500 parts) and air dried to give a black solid (172 parts) which contained manganese (15.5 parts) and cobalt (8.08 parts) and sodium (27.5 parts).

A further wash with hot water produced a black solid containing a considerably reduced sodium content, ie manganese (41.7%) cobalt (21.0%) and sodium (0.33%).

The catalyst metal residues recovered, represented at least about 80% of the original amount present still an approximate manganese/cobalt ratio of 2/1 w/w.

In a second trial nitrogen sparge was used through the neutralisation stage and filtration was carried out in a nitrogen atmosphere. The solid obtained was considerably lighter in colour suggesting formation of manganese dioxide had been reduced.

The mother liquors and combined water washes from the first trial when collected (9000 parts) were found to contain manganese (0.076 parts) equivalent to 8.4 ppm and cobalt (0.044 parts) equivalent to 4.9 ppm, corresponding to 0.4% of the total metal residues originally present.

The catalyst residues were dissolved in the calculated amount of dilute stirred optionally warmed aqueous 48% hydrobromic acid (1:4 $H_2O$). A little bromine was evolved (if desired this can be eliminated by incorporating acetaldehyde or formic acid in the acid). The resulting solution was of suitable purity for reuse in a process for the oxidation of para-xylene to terephthalic acid.

A further sample of the waste by-product stream from terephthalic acid manufacture containing less catalyst (2000 parts, containing manganese (10 parts) and cobalt (6 parts)) subjected to the conditions described previously yielded a black solid (123.8 parts) which contained manganese (10 parts), cobalt (5 parts) and sodium (15.35 parts).

The mother liquors plus first water wash (9000 parts) obtained from the filtration to remove the metallic residues first described were heated to a maximum of 80⁻C with agitation. Sulphuric acid of 96% strength (1500 parts) was added slowly until the reactants reached a pH value of 2.0.

The mass was cooled to room temperature and filtered to remove the precipitated mixed aromatic carboxylic acids. The wet solids contained manganese (2.5 ppm) cobalt (4.6 ppm) and sodium (0.39%).

The wet solid was agitated with cold water (2000 parts) for 1 hr and again filtered. The buff coloured granular solid obtained after air drying (1663 parts) had the following composition:

water 14%
mixed aromatic acids 85.8%
sodium 500 ppm approx.
manganese 4 ppm approx.
cobalt 1.0 ppm

This corresponds to a recovery of about 90% of total aromatic acids.

A buff coloured solid obtained by subjecting a sample of a similar terephthalic acid waste by-product stream from terephthalic acid manufacture to the conditions previously described contained 51% benzoic acid. In addition it contained a mixture of terephthalic acid, isophthalic acid, polycarboxy substituted diphenyls and polyphenyls and traces of p-toluic acid.

The solid (500 parts samples) was slurried with three times its own weight with solvent once or twice as shown in the table. The results are displayed in the following table demonstrating the value of using specific solvents.

TABLE

| Cold solvent | No of washes | Residual benzoic acid in solids (Parts) | Benzoic acid in solvent (Parts) | Other acids in solvent |
|---|---|---|---|---|
| Toluene | 1 | 38.5 | 186.5 | 0.1% |
| Toluene | 2 | 15 | 210 | 0.1% |
| 1,1,1,-trichloro-ethane | 1 | 21.5 | 203.5 | 0.1% |
| 1,1,1,-trichloro-ethane | 2 | 17.5 | 207.5 | 0.1% |
| Methanol | 1 | 34 | 191 | ** iso-phthalic acid |
| Acetone (Hot) | 1 | 15 | 210 | ** iso-phthalic acid |
| Ethylene glycol monobutylether | 1 | 22.5 | 232.5 | 0.1% |
| Ethylene glycol monobutylether | 2 | 5.0 | 250 | 0.1% |
| Ethylene glycol monomethylether | 1 | 5.5 | 249.5 | ** iso-phthalic acid |
| *DMAGS | 1 | 7.5 | 247.5 | 0.1% |

\* Dimethyl esters of mixed adipic, glutaric succinic acids

\*\* Isophthalic acid was present in significant (but not determined) amounts.

A buff coloured solid obtained from subjecting a further sample of similar waste by-product stream from terephthalic acid manufacture to the conditions previously described and containing 40.13% benzoic acid was extracted once with three times its own weight cold toluene.

After filtration and air drying the solids component (representing 57% w/w of the total) contained 4.25% benzoic acid.

Removal of toluene by evaporation from the extract left a solid residue representing 43% w/w of the total which had the following composition.

Benzoic acid 96.06%

p-toluic acid 1.7%

terephthalic acid 1.14%

isophthalic acid 0.85%

The aqueous acetic acid solution of sodium sulphate remaining as mother liquors after the mixed polycarboxylic acid had been removed by filtration can be further treated as follows. Cool to 5°C, remove sodium sulphate crystals by filtration and wash with a small amount of water pre-cooled to 5°C. Wash liquors may be returned to the mother liquors.

The mother liquors can be concentrated initially to remove excess water. Continued distillation yields an acetic acid rich distillate which can be recycled to terephthalic acid manufacture.

Sodium sulphate can be re-crystallised from water to yield purified sodium sulphate.

## Claims

1. Recovery of metal catalysts and other useful products from a process of producing terephthalic acid in which para-xylene is oxidised to terephthalic acid by contacting it in solution in acetic acid with elemental oxygen in the presence of a dissolved catalyst which comprises cobalt and manganese, and separating terephthalic acid as a solid from a mother liquor, by distilling acetic acid from at least part of the mother liquor to leave a residue, dissolving the alkali soluble part of the residue in an aqueous solution of an ammonium or alkali metal hydroxide, carbonate or bicarbonate and thereby leaving a solid deposit of cobalt and manganese compounds, separating the solid deposit, precipitating organic acids by adding acid to the aqueous solution, and separating them, solvent extracting the solid organic acids to remove the monobasic acids and leaving a residue comprising mixed polybasic organic acids.

2. A process as claimed in claim 1 in which the catalyst comprises bromine and in which the solid deposit of cobalt and manganese compounds is reacted with hydrobromic acid and returned to the oxidation step of the process.

3. A process as claimed in claim 1 or 2 in which the acids precipitated from the alkaline solution are washed with water to remove water soluble salts before being solvent extracted.

4. A process as claimed in any preceding claim in which the solvent used in solvent extracting the organic acids comprises toluene, dimethyl succinate, glutarate and/or adipate butoxy or ethoxy ethanol, carbon tetrachloride, chloroform, 1,1,1 - trichlorethane or ethylene glycol.

## Revendications

1. Récupération de catalyseurs métalliques et d'autres produits utiles provenant d'un procédé de production d'acide téréphtalique dans lequel du p-xylène est oxydé en acide téréphtalique par mise en contact du p-xylène en solution dans l'acide acétique avec de l'oxygène élémentaire en présence d'un catalyseur dissous qui comprend du cobalt et du manganèse, et séparation de l'acide téréphtalique sous forme de solide à partir d'une liqueur-mère, caractérisée en ce qu'elle comprend la distillation d'acide acétique à partir d'au moins une partie de la liqueur-mère pour laisser un résidu, la dissolution de la partie soluble dans un alcalin du résidu dans une solution aqueuse d'un hydroxyde, carbonate ou carbonate acide d'ammonium ou de métal alcalin de façon à laisser un dépôt solide de composés de cobalt et de manganèse, la séparation du dépôt solide, la précipitation des acides organiques par addition d'acide à la solution aqueuse et leur séparation, l'extraction par solvant des acides organiques solides pour éliminer les monoacides et laisser un résidu comprenant des polyacides organiques.

2. Procédé suivant la revendication 1, caractérisé en ce que le catalyseur comprend du brome et que le dépôt solide de composés de cobalt et de manganèse est traité avec de l'acide bromhydrique et renvoyé vers l'étape d'oxydation du procédé.

3. Procédé suivant les revendications 1 ou 2, caractérisé en ce que les acides précipités à partir de la solution alcaline sont lavés à l'eau pour éliminer des sels solubles dans l'eau avant extraction par un solvant.

4. Procédé suivant l'une quelconque des revendications précédentes, caractérisé en ce que le solvant utilisé dans l'extraction par un solvant des acides organiques, comprend le toluène, le succinate, le glutarate et/ou l'adipate de diméthyle, le butoxy ou l'éthoxy étha nol, tétrachlorure de carbone, le chloroforme, le 1,1,1-trichloréthane ou l'éthylèneglycol.

**Ansprüche**

1. Rückgewinnung von Metallkatalysatoren und anderen nutzbaren Produkten aus einem Verfahren zur Herstellung von Terephthalsäure, bei dem para-Xylol zu Terephthalsäure oxidiert wird, indem es in Form einer Lösung in Essigsäure in Gegenwart eines gelösten Katalysators, der Cobalt und Mangan enthält, mit elementarem Sauerstoff in Berührung gebracht wird, und von einer Mutterlauge Terephthalsäure als Feststoff abgetrennt wird, durch Abdestillieren von Essigsäure von wenigstens einem Teil der Mutterlauge, wobei ein Rückstand zurückgelassen wird, Auflösen des alkalilöslichen Teils des Rückstands in einer wäßrigen Lösung eines Ammonium- oder Alkalimetallhydroxids, -carbonats oder -hydrogencarbonats, wodurch ein fester Niederschlag aus Cobalt- und Manganverbindungen zurückgelassen wird, Abtrennen des festen Niederschlags, Ausfällen organischer Säuren durch Zusatz von Säure zu der wäßrigen Lösung und Abtrennen der organischen Säuren, Lösungsmittelextraktion der festen organischen Säuren zum Abtrennen der einbasigen Säuren und Zurücklassen eines Rückstands, der gemischte mehrbasige organische Säuren enthält.

2. Verfahren nach Anspruch 1, bei dem der Katalysator Brom enthält und bei dem der feste Niederschlag aus Cobalt- und Manganverbindungen mit Bromwasserstoffsäure umgesetzt und zu dem Oxidationsschritt des Verfahrens zurückgeführt wird.

3. Verfahren nach Anspruch 1 oder 2, bei dem die Säuren, die aus der alkalischen Lösung ausgefällt worden sind, zur Entfernung wasserlöslicher Salze mit Wasser gewaschen werden, bevor sie der Lösungsmittelextraktion unterzogen werden.

4. Verfahren nach einem der vorhergehenden Ansprüche, bei dem das Lösungsmittel, das bei der Lösungsmittelextraktion der organischen Säuren verwendet wird, Toluol, Dimethylsuccinat, -glutarat und/oder -adipat, Butoxy- oder Ethoxyethanol, Kohlenstofftetrachlorid, Chloroform, 1,1,1-Trichlorethan oder Ethylenglykol enthält.